# EUROPÄISCHE PATENTSCHRIFT

(11) **EP 0 505 965 B1**
(45) Veröffentlichungstag und Bekanntmachung des Hinweises auf die Patenterteilung: **26.10.1994**
(21) Anmeldenummer: 92104994.6
(22) Anmeldetag: 23.03.1992
(51) Int. Cl.: C07C 315/04, C07C 317/44

(54) **Verfahren zur Herstellung von Alkansulfonylbenzoesäuren**
Process for the preparation of alkanesulfonyl benzoic acids
Procédé pour la préparation d'acides alkanesulfonyl benzoiques

(30) Priorität: 26.03.1991 DE 4109819
(43) Veröffentlichungstag der Anmeldung: 30.09.1992
(73) Patentinhaber: HOECHST AKTIENGESELLSCHAFT, 65926 Frankfurt am Main (DE)
(72) Erfinder: Röhrscheid, Freimund, Dr., W-6233 Kelkheim (Taunus) (DE)

(56) Entgegenhaltungen:
- WO-A-90/13537
- CHEMICAL ABSTRACTS, vol. 111, no. 15, 9. Oktober 1989, Columbus, Ohio, US; H. SANAMI et al, "Purification of diphenyl sulfone-tetracarboxylic acids by recrystallization", abstract no. 133781, Seite 722; & JP-A-1 079 145

## Beschreibung

Die Erfindung betrifft ein verbessertes Verfahren zur Herstellung von Alkansulfonylbenzoesäuren aus den entsprechenden Alkansulfonyl-alkyl-benzolen mit molekularem Sauerstoff in Essig- und/oder Propionsäure.

Alkansulfonylbenzoesäuren sind Vorprodukte für Schädlingsbekämpfungsmittel und Herbizide (US-PS 4,704,467, EP-OS 203 428).

In der WO 90/13537 wird ein Verfahren zur Oxidation von gegebenenfalls substituierten Alkansulfonyl-alkyl-benzolen mit molekularem Sauerstoff in Essig- oder Propionsäure zu den entsprechenden Alkansulfonylbenzoesäuren beschrieben. Die Oxidation wird in Gegenwart eines in der Saure löslichen Katalysators, der Kobalt- und Bromidionen und gegebenenfalls Manganionen enthält, unter erhöhtem Druck bei Temperaturen oberhalb von 120°C durchgeführt.

Die Alkansulfonylbenzoesäuren kristallisieren je nach Konzentration beim Abkühlen aus der Reaktionslösung aus und werden durch Filtration abgetrennt. Im Filtrat befinden sich der Katalysator und erhebliche Mengen an gelöstem Produkt. Deshalb wird in einer bevorzugten Ausführungsform das während der Reaktion entstandene Wasser aus dem Filtrat abgetrennt und diese Mutterlauge wieder als Reaktionsmedium für die nächste Oxidation verwendet.

Aufgabe der vorliegenden Erfindung war es daher, ein Verfahren zur Herstellung von Alkansulfonylbenzoesäuren bereitzustellen, das insbesondere Alkansulfonylbenzoesäuren in hoher Ausbeute und Reinheit liefert und eine unproblematische Wiederverwendung der Mutterlauge ermöglicht.

Es wurde nun überraschend gefunden, daß sich Alkansulfonylbenzoesäuren in einfacher Weise aus Alkansulfonyl-alkyl-benzolen der allgemeinen Formel I (siehe Patentanspruch 1), worin R¹ und R² gleich oder verschieden und Alkyl mit 1 bis 4 C-Atomen sind, R² jedoch eine andere Bedeutung als t-Butyl hat, und X für H, F, Cl, Br oder NO₂ steht, mit molekularem Sauerstoff in Essig- und/oder Propionsäure in Gegenwart eines Katalysators, der Kobalt- und Bromionen und, insbesondere wenn R² eine andere Bedeutung als Methyl hat, auch Manganionen enthält, herstellen lassen, wenn man die Reaktion zusätzlich in Gegenwart von Metallionen der 2. und/oder 3. Hauptgruppe durchführt.

Die Metallionen werden bevorzugt in Form der Salze der betreffenden Carbonsäure zugefügt. Das Bromid kann in Form einer HBr-Lösung oder zweckmäßig als Magnesium-, Kalzium-, Kobalt- oder Manganbromid zugegeben werden. Als Metallionen der 2. und 3. Hauptgruppe kommen insbesondere solche von Magnesium, Kalzium, Strontium, Barium und Aluminium in Frage, bevorzugt jedoch Mg²⁺ und besonders Ca²⁺.

Durch Verwendung von Kalziumazetat bei der Oxidation von 2-Chlor-4-methansulfonyltoluol (vgl. Beispiele) kann die Mutterlauge bei gleichbleibender Katalysatoraktivität z.B. neunmal wiederverwendet werden. Das Produkt ist nach dem Waschen mit Eisessig farblos und kann mit Wasser gewaschen werden, ohne daß Kobalt- oder Manganionen ins Abwasser gelangen.

Zu Beginn einer jeden Oxidation beträgt das Molverhältnis der Summe der Metallionen der 2. und/oder 3. Hauptgruppe zu der Summe an Kobalt- und gegebenenfalls Manganionen zweckmäßig (0,1 bis 10):1, vorzugsweise (0,3 bis 3):1, und insbesondere (0,5 bis 1,5):1, wobei die Menge an Metallionen der 2. und/oder 3. Hauptgruppe zweckmäßig von 1 bis 5 Mol-% des Alkansulfonylalkyl-benzols beträgt.

Als Alkylgruppen für R¹ und R² kommen jeweils Methyl, Äthyl, n- und iso-Propyl, n-, sek- und iso-Butyl in Frage, für R¹ zusätzlich auch t-Butyl. Bei der Oxidation wird der Rest R² zur COOH-Gruppe oxidiert. Zur Oxidation der Methylgruppe R² sind 1,5 Mol Sauerstoff erforderlich.

Die Ausgangsverbindungen sind leicht zugänglich; z.B. lassen sich Alkansulfonyltoluole aus Toluolsulfonsaure durch Reduktion zum toluolsulfinsauren Natrium und durch Umsetzung mit Dimethylsulfat oder Diäthylsulfat herstellen.

Das vorliegende Verfahren wird vorteilhaft unter den für die Oxidation von Alkylgruppen besonders wirksamen Bedingungen, wie sie z.B. für die Oxidation beider Methylgruppen des p-Xylols zur Terephthalsäure angewendet werden, durchgeführt. Durch Anwendung dieser sehr wirksamen Oxidationsbedingungen ist es möglich, die Alkylgruppe am Benzolring zu oxidieren, auch wenn der Benzolring zusätzlich zur stark elektronenziehenden Alkansulfonylgruppe einen zusätzlichen desaktivierenden Substituenten X trägt. Es ist jedoch überraschend, daß unter diesen Oxidationsbedingungen nur die Alkylgruppe am Benzolring oxidiert wird, die Alkansulfonylgruppe dagegen erhalten bleibt. So wird im Gegensatz zur Alkansulfonylgruppe z.B. die Acetylgruppe des Acetophenons unter diesen Bedingungen leicht oxidativ abgebaut.

Die Essig- und/oder Propionsäure werden im allgemeinen in wasserfreier Form eingesetzt; jedoch bildet sich bei der Oxidation Wasser. Das Reaktionssystem enthält jedoch im allgemeinen nicht mehr als 15 % Wasser, insbesondere höchstens 5 %. Die Verwendung von wasserfreier Essigsäure ist bevorzugt.

Die Anwesenheit von Manganionen ist für die gewünschte Oxidation der am Aromaten gebundenen Äthyl-, Propyl- und Butylgruppe notwendig, aber auch für die Oxidation der Methylgruppe zweckmäßig, wenngleich für diese letztere auch eine Kombination aus Metallionen der 2. und/oder 3. Hauptgruppe, Kobalt- und Bromionen genügt. Die Gegenwart von Manganionen ermöglicht eine Verringerung der erforderlichen Kobaltmenge bei gleicher Aktivität des Katalysators.

Das Verhältnis der Konzentration von Kobalt- zu Manganionen beträgt im allgemeinen 1:(0,2 bis 3), bevorzugt 1:(0,3 bis 1,2). Das Verhältnis der Summe der Konzentration der Kobalt- und Manganionen zu Bromionen beträgt zweckmäßig 1:(0,01 bis 2), vorzugsweise 1:(0,1 bis 1), besonders bevorzugt 1:(0,2 bis 0,7). Die Summe der Konzentrationen von Kobalt- und Manganionen liegt zweckmäßig im Bereich von 0,01 bis 0,2 Mol, vorzugsweise von 0,02 bis 0,15 Mol und insbesondere von 0,04 bis 0,12 Mol Metallionen je l der flüssigen Phase.

Der molekulare Sauerstoff wird bevorzugt in Form trockener Luft in die flüssige Phase des Reaktors eingeführt. Das Verfahren wird zweckmäßig unter einem Sauerstoffpartialdruck von 1,5 bis 8, vorzugsweise von 2,4 bis 7 und insbesondere von 2,8 bis 6 bar durchgeführt. Die Reaktionstemperatur liegt zweckmäßig im Bereich von 120 bis 220°C, vorzugsweise von 130 bis 180°C, und insbesondere von 135 bis 160°C.

Für die vollständige und rasche Oxidation der Alkylgruppe am Benzolring ist es ganz besonders vorteilhaft, bei einer Katalysatorkonzentration von 0,04 bis 0,12 Mol an Kobalt- und Manganionen pro l flüssiger Phase zu arbeiten und an der Eintrittsstelle in die flüssige Phase einen hohen Sauerstoffpartialdruck von mindestens 2,4 bis 2,5 bar anzuwenden. Die Kombination aus diesen beiden Maßnahmen ermöglicht es auch, die erforderliche Reaktionstemperatur niedrig zu halten. Wenn man keine extrem scharfen Bedingungen kombiniert, wird die Alkansulfonylgruppe nur unerheblich oxidativ angegriffen.

Die Alkansulfonylsäuren kristallisieren je nach Konzentration beim Abkühlen aus der Reaktionslösung aus und können durch Filtration abgetrennt werden. Im Filtrat befinden sich aber oft erhebliche Mengen an gelöstem Produkt und der gesamte Katalysator. Eine vorteilhafte Ausführungsform des Verfahrens besteht deshalb darin, aus dem Filtrat an einer Kolonne das während der Reaktion entstandene Wasser abzutrennen und diese Mutterlauge wieder als Reaktionsmedium für die nächste Oxidation zu verwenden. Dadurch wird Katalysator eingespart, das Abwasser nicht unnötig belastet und die isolierte Ausbeute des darauffolgenden Ansatzes ist höher.

In den folgenden Beispielen steht "OAc" für Acetat.

### Beispiele

1. In einem 1 l-Autoklaven aus Edelstahl, der mit Thermometer, Rührer, Rückflußkühler und Druckhalteventil ausgestattet war, wurde eine Mischung aus 250 g 2-Chlor-4-methansulfonyltoluol, 15 g Co(OAc)₂·4H₂O, 10 g Ca(OAc)₂, 2 g einer 62 %igen wäßrigen Bromwasserstoff-Lösung und 455 g Eisessig unter 16 bar Stickstoff auf 150°C erhitzt. Dann wurde Luft (16 bar) über ein Tauchrohr in die flüssige Phase eingeleitet. Die exotherme Reaktion setzte sofort ein, und durch Kühlung wurde die Temperatur bei 155 bis 160°C gehalten. Durch Regelung der Luftzufuhr wurde der Sauerstoffgehalt im Abgas bei 5 bis 6 Vol.-% gehalten. Nach Ende der exothermen Reaktion wurde die heiße Reaktionslösung entnommen und unter Rühren auf 20°C abgekühlt. Die ausgefallenen Kristalle wurden abgesaugt, vier Mal mit je 50 ml Eisessig und drei Mal mit je 75 ml Wasser gewaschen und bei 80°C und 65 mbar im schwachen Luftstrom getrocknet.
   - Ausbeute:: 248,0 g (86,5 % d.Th.) 2-Chlor-4-(methansulfonyl)-benzoesäure; Fp 193 bis 194°C.
2. Das Filtrat und die beim Waschen mit Essigsäure erhaltene Flüssigkeit aus Beispiel 1 wurden unter Atmosphärendruck in einer Kolonne abdestilliert. Es wurde zuerst das Wasser abgetrennt, dann wurde so viel Essigsäure abdestilliert, bis ca. 440 g Lösung im Destillationskolben zurückblieben. Die Temperatur des Sumpfes betrugt 121°C.
   Im 1 l-Oxidationsautoklaven wurde eine Mischung aus 204,7 g 2-Chlor-4-methansulfonyl-toluol, 2,3 g Ca(OAc)₂, 1,9 g 62 %ige wäßrige HBr-Lösung und der aufkonzentrierten Mutterlauge (440 g) vorgelegt. Die Oxidation und Aufarbeitung erfolgte wie im Beispiel 1.
   - Ausbeute:: 220,7 g (94,1 % d.Th.) 2-Chlor-4-(methansulfonyl)-benzoesäure, Fp. 192 bis 193°C.
3. bis 10. Das Beispiel 2 wurde acht Mal jeweils mit der entwässerten Mutterlauge des vorhergehenden Ansatzes wiederholt.
   Die durchschnittliche Ausbeute in den Beispielen 3 bis 10 betrug 219,7 g (93,6 % d.Th.), Fp. 191 bis 193°C.

## Patentansprüche

1. Verfahren zur Herstellung von Alkansulfonylbenzoesäuren aus Alkansulfonylalkyl-benzolen der Formel 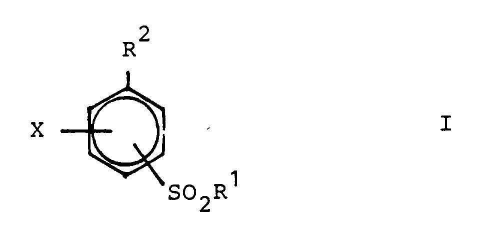 worin R¹ und R² gleich oder verschieden und Alkyl mit 1 bis 4 C-Atomen sind, R² jedoch eine andere Bedeutung als t-Butyl hat, und X für H, F, Cl, Br oder NO₂ steht, mit molekularem Sauerstoff in Essig- und/oder Propionsäure in Gegenwart eines Katalysators, der Kobalt- und Bromionen und, insbesondere wenn R² eine andere Bedeutung als Methyl hat, auch Manganionen enthält, dadurch gekennzeichnet, daß zusätzlich in Gegenwart von Metallionen der 2. und/oder 3. Hauptgruppe gearbeitet wird.

2. Verfahren nach Anspruch 1, dadurch gekennzeichnet, daß Essigsäure verwendet wird.

3. Verfahren nach Anspruch 1 oder 2, dadurch gekennzeichnet, daß als Metallionen der 2. und/oder 3. Hauptgruppe solche von Magnesium, Kalzium, Strontium, Barium oder Aluminium, bevorzugt Mg²⁺ und/oder Ca²⁺, eingesetzt werden.

4. Verfahren nach Anspruch 3, dadurch gekennzeichnet, daß das Ca²⁺ in Form von Kalziumazetat eingebracht wird.

5. Verfahren nach einem oder mehreren der Ansprüche 1 bis 4, dadurch gekennzeichnet, daß das Molverhältnis der Summe der Metallionen der 2. und/oder 3. Hauptgruppe und der Summe an Kobalt- und/oder Maganionen (0,1 bis 10):1, vorzugsweise (0,3 bis 3):1 und insbesondere (0,5 bis 1,5):1 beträgt.

6. Verfahren nach Anspruch 5, dadurch gekennzeichnet, daß die Menge an Metallionen der 2. und/oder 3. Hauptgruppe von 1 bis 5 Mol-% des Alkansulfonylalkyl-benzols beträgt.

7. Verfahren nach einem oder mehreren der Ansprüche 1 bis 6, dadurch gekennzeichnet, daß Kobalt- und Manganionen im Konzentrationsverhältnis 1:(0,2 bis 3), vorzugsweise 1:(0,3 bis 1,2), verwendet werden.

8. Verfahren nach einem oder mehreren der Ansprüche 1 bis 7, dadurch gekennzeichnet, daß das Verhältnis der Summe der Konzentrationen von Kobalt- und Manganionen und der Konzentration der Bromionen 1:(0,01 bis 2), vorzugsweise 1:(0,1 bis 1) und insbesondere 1:(0,2 bis 0,7) beträgt.

9. Verfahren nach einem oder mehreren der Ansprüche 1 bis 8, dadurch gekennzeichnet, daß die Summe der Konzentrationen von Kobalt- und Manganionen 0,01 bis 0,2 Mol, vorzugsweise 0,02 bis 0,15 Mol, besonders bevorzugt 0,04 bis 0,12 Mol Metallionen je l flüssiger Phase beträgt.

10. Verfahren nach einem oder mehreren der Ansprüche 1 bis 9, dadurch gekennzeichnet, daß der Sauerstoffpartialdruck im Reaktor 1,5 bis 8, vorzugsweise 2,4 bis 7 und insbesondere 2,8 bis 6 bar beträgt.

11. Verfahren nach einem oder mehreren der Ansprüche 1 bis 10, dadurch gekennzeichnet, daß die Reaktionstemperatur 120 bis 220°C, vorzugsweise 130 bis 180°C und insbesondere 135 bis 160°C beträgt.

12. Verfahren nach einem oder mehreren der Ansprüche 1 bis 11, dadurch gekennzeichnet, daß ausgefallenes kristallines Produkt abgetrennt und die Mutterlauge erneut als Reaktionsmedium eingesetzt wird.

## Claims

1. A process for the preparation of alkanesulfonylbenzoic acids from alkanesulfonylalkylbenzenes of the formula 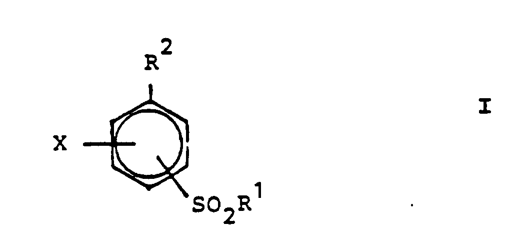 in which R¹ and R² are identical or different and are alkyl having 1 to 4 carbon atoms, but R² has a meaning other than t-butyl, and X is H, F, Cl, Br or NO₂, using molecular oxygen in acetic acid and/or propionic acid in the presence of a catalyst containing cobalt ions and bromine ions and, in particular when the meaning of R² is other than methyl, also manganese ions, which is additionally carried out in the presence of metal ions of Main Group 2 and/or 3.

2. The process as claimed in claim 1, wherein acetic acid is used.

3. The process as claimed in claim 1 or 2, wherein magnesium, calcium, strontium, barium or aluminum ions, preferably Mg²⁺ and/or Ca²⁺, are employed as metal ions of Main Group 2 and/or 3.

4. The process as claimed in claim 3, wherein the Ca²⁺ is introduced in the form of calcium acetate.

5. The process as claimed in one or more of claims 1 to 4, wherein the molar ratio of the total of the metal ions of Main Group 2 and/or 3 and the total of cobalt ions and/or manganese ions is (0.1 to 10):1, preferably (0.3 to 3):1 and, in particular, (0.5 to 1.5):1.

6. The process as claimed in claim 5, wherein the amount of metal ions of Main Group 2 and/or 3 is 1 to 5 mol% of the alkanesulfonylalkylbenzene.

7. The process as claimed in one or more of claims 1 to 6, wherein cobalt ions and manganese ions are used in a concentration ratio of 1:(0.2 to 3), preferably 1:(0.3 to 1.2).

8. The process as claimed in one or more of claims 1 to 7, wherein the ratio of the total of the concentrations of cobalt ions and manganese ions and the concentration of the bromine ions is 1:(0.01 to 2), preferably 1:(0.1 to 1) and, in particular, 1:(0.2 to 0.7).

9. The process as claimed in one or more of claims 1 to 8, wherein the total of the concentrations of cobalt ions and manganese ions is 0.01 to 0.2 mol, preferably 0.02 to 0.15 mol, particularly preferably 0.04 to 0.12 mol, of metal ions per l of liquid phase.

10. The process as claimed in one or more of claims 1 to 9, wherein the oxygen partial pressure in the reactor is 1.5 to 8, preferably 2.4 to 7 and, in particular, 2.8 to 6 bar.

11. The process as claimed in one or more of claims 1 to 10, wherein the reaction temperature is 120 to 220°C, preferably 130 to 180°C and, in particular, 135 to 160°C.

12. The process as claimed in one or more of claims 1 to 11, wherein a crystalline product which has precipitated is separated off and the mother liquor is recycled as reaction medium.

## Revendications

1. Procédé de préparation d'acides alcane sulfonyl benzoïques à partir d'alcane sulfonyl alkyle benzènes de formule : 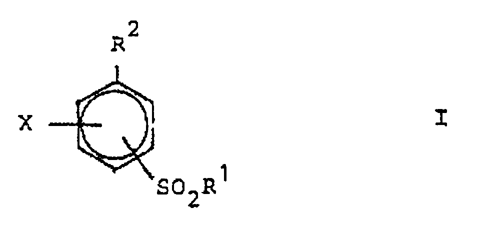 (dans laquelle R¹ et R² sont identiques ou différents et représentent chacun un groupe alkyle ayant 1 à 4 atomes de carbone; R² pouvant cependant avoir un autre sens qu'un groupe t-butyle; et X représente H, F, Cl, Br ou NO₂), par réaction avec de l'oxygène moléculaire dans de l'acide acétique et/ou propionique, en présence d'un catalyseur contenant des ions cobalt et brome, et en particulier, lorsque R² a un autre sens qu'un groupe méthyle, contenant également des ions manganèse, procédé caractérisé en ce qu'on travaille en outre en présence d'ions de métaux du second et/ou du troisième groupe principal, du Tableau Périodique des Eléments].

2. Procédé selon la revendication 1, caractérisé en ce qu'on utilise de l'acide acétique.

3. Procédé selon la revendication 1 ou 2, caractérisé en ce qu'on utilise comme ions de métaux du groupe principal 2 et/ou 3 [du Tableau Périodique des Eléments] des ions magnésium, calcium, strontium, baryum ou aluminium, de préférence Mg²⁺ et/ou Ca²⁺.

4. Procédé selon la revendication 3, caractérisé en ce qu'on introduit l'ion Ca²⁺ sous forme d'acétate de calcium.

5. Procédé selon une ou plusieurs des revendications 1 à 4, caractérisé en ce que le rapport molaire de la somme des ions de métaux du second et/ou du troisième groupe principal à la somme des ions cobalt et/ou manganèse vaut (0,1 à 10):1, avantageusement (0,3 à 3) :1 et notamment (0,5 à 1,5):1.

6. Procédé selon la revendication 5, caractérisé en ce que la quantité des ions de métaux du second et/ou du troisième groupe principal vaut 1 à 5 moles % de l'alcane sulfonyl benzène.

7. Procédé selon une ou plusieurs des revendications 1 à 6, caractérisé en ce qu'on utilise des ions cobalt et manganèse en un rapport entre les concentrations valant 1:(0,2 à 3), avantageusement 1:(0,3 à 1,2).

8. Procédé selon une ou plusieurs des revendications 1 à 7, caractérisé en ce que le rapport entre la somme des concentrations des ions cobalt et manganèse et la concentration des ions brome vaut 1:(0,01 à 2), avantageusement 1:(0,1 à 1) et notamment 1:(0,2 à 0,7).

9. Procédé selon une ou plusieurs des revendications 1 à 8, caractérisé en ce que la somme des concentrations des ions cobalt et manganèse vaut 0,01 à 0,2 mole, avantageusement 0,2 à 0,15 mole, de façon particulièrement préférée 0,04 à 0,12 mole d'ions de métaux par litre de la phase liquide.

10. Procédé selon une ou plusieurs des revendications 1 à 9, caractérisé en ce que la pression partielle de l'oxygène dans le réaction vaut 1,5 à 8, avantageusement 2,4 à 7 et notamment 2,8 à 6 bars.

11. Procédé selon une ou plusieurs des revendications 1 à 10, caractérisé en ce que la température de réaction vaut 120 à 220°C, avantageusement 130 à 180°C et notamment 135 à 160°C.

12. Procédé selon une ou plusieurs des revendications 1 à 11, caractérisé en ce que le produit cristallin précipité est séparé et la liqueur mère est utilisée à nouveau comme milieu pour la réaction.
